# EUROPÄISCHE PATENTSCHRIFT

(11) **EP 2 354 142 B1**
(45) Veröffentlichungstag und Bekanntmachung des Hinweises auf die Patenterteilung: **31.07.2013**
(21) Anmeldenummer: 11003589.6
(22) Anmeldetag: 29.05.2008
(51) Int. Cl.: C07D 475/04

(54) **Verfahren zur Herstellung des stabilen, amorphen Calciumsalzes von (6S)-N(5)-Methyl-5,6,7,8-tetrahydrofolsäure**
Process for the preparation of the stable, amorphous calcium salt of (6S)-N(5)-methyl-5,6,7,8-tetrahydrofolic acid
Procédé pour la préparation du sel de calcium de l'acide (6S)- N(5)-méthyl-5,6,7,8-tétrahydrofolique amorphe stable

(30) Priorität: 30.05.2007 CH 8562007
(43) Veröffentlichungstag der Anmeldung: 10.08.2011
(62) Teilanmeldung aus: 08748372.3
(73) Patentinhaber: Cerbios-Pharma S.A., CH-6917 Barbengo (CH)
(72) Erfinder: Manzotti, Raffaella, 22020 Pellio Intelvi (Como) (IT); Morosoli, Moreno, 6950 Tesserete (CH)
(74) Vertreter: Zink-Wild, Markus Peter

(56) Entgegenhaltungen:
- EP-A- 0 455 013
- EP-A- 1 044 975

## Beschreibung

Die vorliegende Erfindung betrifft ein Verfahren zur Herstellung des stabilen, amorphen Calciumsalzes von (6S)-N(5)-Methyl-5,6,7,8-tetrahydrofolsäure.

In der vorliegenden Erfindung wird für 5,6,7,8-Tetrahydrofolsäure manchmal die Abkürzung THF verwendet.

Eine kurze Zusammenfassung über die pharmakologische Bedeutung von N(5)-Methyl-5,6,7,8-tetrahydrofolsäure - hierin teilweise mit N(5)-Methyl-THF abgekürzt - und Derivaten davon ist in der Beschreibungseinleitung von EP 0 455 013 A1 gegeben. Im gleichen Dokument wird auch auf die Bedeutung der individuellen (6S)-und (6R) -Diastereoisomeren der N(5)-Methyl-THF hingewiesen. Ebenso wird hierin der Stand der Technik betreffend die Herstellung der reinen (6S)- und (6R)-Diastereoisomeren der N(5)-Methyl-THF beschrieben.

In EP 1 044 975 A1 werden stabile kristalline Salze von N(5)-Methyl-THF beschrieben. Ebenso werden hierin auch kristalline Calciumsalze von (6S)-N(5)-Methyl-THF beschrieben, wobei diese Salze im entsprechenden Pulverröntgendiagramm klar definierte 2-Theta-Werte haben.

Beim Herstellungsverfahren dieser Salze werden als Ausgangsmaterialien entweder das (6RS)-Diastereoisomerengemisch oder die bereits aufgetrennten (6S)-oder (6R)-Diastereoisomeren verwendet. Dieses Verfahren beinhaltet eine Temperaturbehandlung von über 60°C, vorzugsweise von über 85°C, wobei in den Ausführungsbeispielen Temperaturen von 90°C bis 100°C erwähnt sind.

Eine solche Temperaturbehandlung ist selbstverständlich für eine industrielle Herstellung dieser Salze wenig geeignet.

In EP 1 044 975 A1 ist nicht angegeben, wie die einzelnen (6S)- oder (6R)-Diastereoisomeren von N(5)-Methyl-THF erhalten worden sind.

Es ist ein Ziel der vorliegenden Erfindung, ein industriell anwendbares Verfahren zur Herstellung eines stabilen Calciumsalzes von (6S)-N(5)-Methyl-5,6,7,8-tetrahydrofolsäure der Formel V zur Verfügung zu stellen.

Das mit diesem Verfahren erhältliche Produkt soll eine Diastereoisomerenreinheit von wenigstens 99 % haben.

Dieses Verfahren soll einfach sein und soll insbesondere keine thermische Behandlung beinhalten.

Mit der vorliegenden Erfindung werden diese Ziele erreicht.

Das erfindungsgemässe Verfahren zur Herstellung einer wässrigen Lösung des Calciumsalzes von (6S)-N(5)-Methyl-5,6,7,8-tetrahydrofolsäure der Formel III,

wobei man (6S)-5,6,7,8-Tetrahydrofolsäure der Formel II mit einem Anteil des entsprechenden (6R)-Diastereoisomers im Bereich von 4 Gew.-% bis 8 Gew.-% in Wasser methyliert,
ist dadurch gekennzeichnet, dass man
- zum erhaltenen methylierten Reaktionsgemisch von 0,70 bis 0,82 Äquivalente, bezogen auf die eingesetzte Menge an THF, an Calciumchlorid hinzugibt,
- aus der erhaltenen wässrigen Lösung das Calciumsalz von (6RS)-N(5)-Methyl-5,6,7,8-tetrahydrofolsäure selektiv auskristallisiert und abtrennt, und
- die wässrige Lösung des Calciumsalzes von (6S)-N(5)-Methyl-5,6,7,8-tetrahydrofolsäure der Formel III mit einem Anteil des entsprechenden (6R)-Diastereo-isomers von ≤ 2 Gew.-% gewinnt.

Eine andere Variante des erfindungsgemässen Verfahrens zur Herstellung des stabilen, amorphen Calciumsalzes von (6S)-N(5)-Methyl-5,6,7,8-tetrahydrofolsäure der Formel V
ist dadurch gekennzeichnet, dass man
- kristalline (6S)-N(5)-Methyl-5,6,7,8-tetrahydrofolsäure der Formel IV in Wasser suspendiert, wobei das Wasser eine Temperatur von 35°C bis 41°C hat,
wobei man die kristalline (6S)-N(5)-Methyl-5,6,7,8-tetrahydrofolsäure der Formel IV gemäss einem Verfahren herstellt, bei dem man
- zu einer wässrigen Lösung des Calciumsalzes von (6S)-N(5)-Methyl-5,6,7,8-tetrahydrofolsäure der Formel III mit einem Anteil des entsprechenden (6R)-Dia-stereoisomers von ≤ 2 Gew.-% entweder Essigsäure oder eine Sulfonsäure in einer solchen Menge hinzugibt, bis ein pH-Wert von 5,5 erhalten wird,
- die so erhaltene Lösung auf eine Temperatur im Bereich von 44°C bis 46°C erwärmt,
- zu dieser erwärmten Lösung entweder Essigsäure oder eine Sulfonsäure in einer solchen Menge hinzugibt, bis ein pH-Wert im Bereich von 4,3 bis 4,4 erhalten wird, wobei die Kristallisation von (6S)-N(5)-Methyl-5,6,7,8-tetrahydrofolsäure der Formel IV beginnt, wobei während dieser Kristallisation der pH-Wert im Bereich von 4,3 bis 4,4 durch kontinuierliche Hinzugabe von entweder Essigsäure oder einer Sulfonsäure aufrecht erhalten wird, und
- den so erhaltenen kristallinen Festkörper bei einer Temperatur im Bereich von 44°C bis 46°C abfiltriert und isoliert,
- zu dieser Suspension eine NaOH Lösung in einer solchen Menge portionenweise hinzugibt, bis ein pH-Wert im Bereich von 6,7 bis 6,9 erhalten wird,
- zur so hergestellten Lösung 0,90 Äquivalente an Calciumchlorid, bezogen auf die eingesetzte Menge an Verbindung der Formel IV, hinzugibt,
- in der so hergestellten Lösung die Konzentration an Verbindung der Formel IV entweder durch Hinzugabe oder durch Entfernung von Wasser auf einen Wert im Bereich von 14 Gew.-% bis 16 Gew.-% einstellt,
- die Fällung des Calciumsalzes von (6S)-N(5)-Methyl-5,6,7,8-tetrahydrofolsäure der Formel V durch die Hinzugabe einer kleinen Menge an vorgängig hergestellter Verbindung der Formel V startet,
- das so hergestellte Gemisch während 1 Stunde bei einer Temperatur von 40°C hält,
- danach innerhalb von 2 Stunden die Temperatur von 40°C auf eine Temperatur von 23°C senkt,
- die genannte Temperatur von 23°C während 16 bis 18 Stunden aufrecht erhält, und
- den erhaltenen Festkörper isoliert.

Bevorzugte Ausführungsformen der vorliegenden Erfindung sind in den abhängigen Ansprüchen definiert.

Im folgenden Teil werden mögliche Ausführungsformen der vorliegenden Erfindung beschrieben.

Dabei wird auch Bezug auf die Figuren genommen.

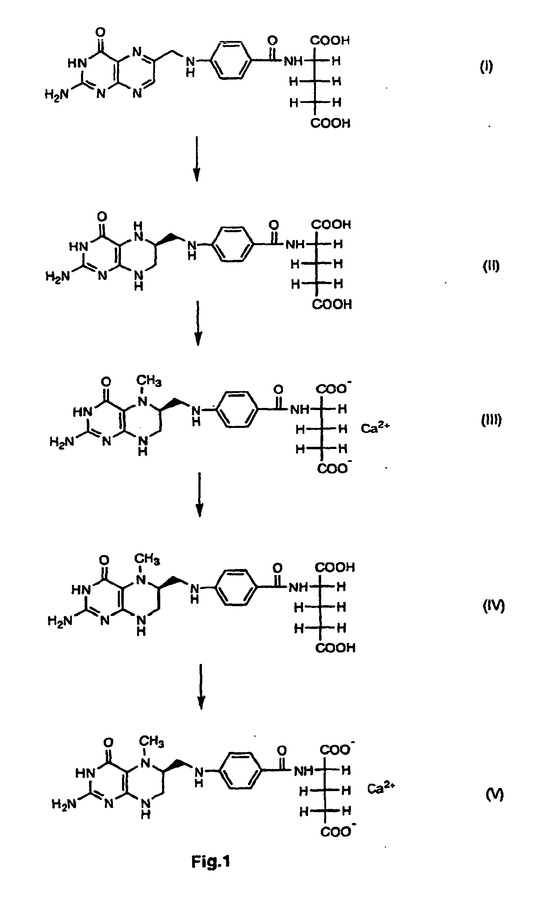

Figur 1 zeigt ein Reaktionsschema, beginnend mit der Verbindung der Formel I und endend mit der Verbindung der Formel V.

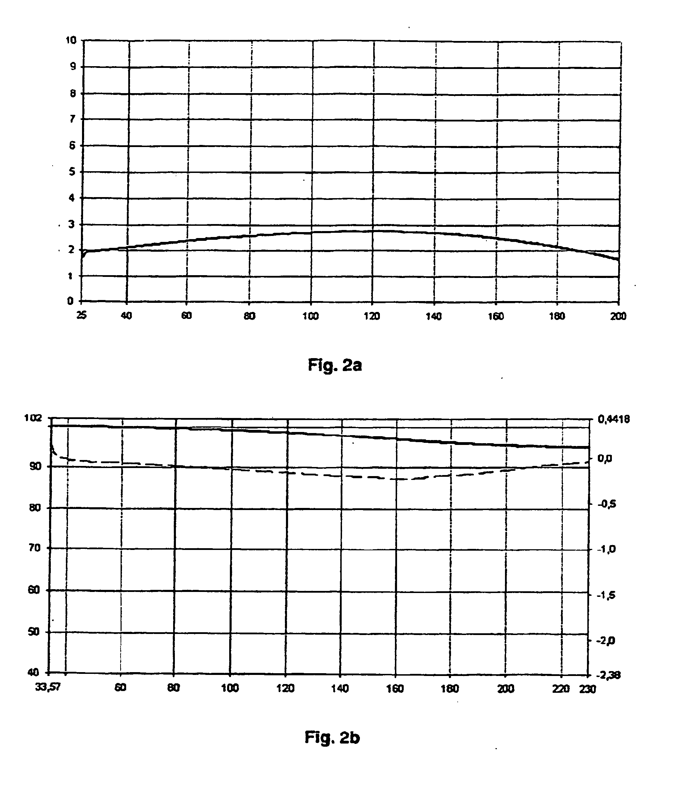

Figur 2a zeigt das "Differential Scanning Calorimetric" Profil, DSC, der Verbindung der Formel IV. Auf der Abszisse ist die Temperatur in °C aufgetragen, und auf der Ordinate ist der endotherme Wärmefluss in mW aufgetragen.
Figur 2b zeigt das thermogravimetrische Profil, TGA, der Verbindung der Formel IV. Auf der Abszisse ist die Temperatur in °C aufgetragen. Auf der linken Ordinate sind Gewichtsprozente und auf der rechten Ordinate ist die Ableitung dieser Gewichtsprozente gegenüber der Zeit t (Minuten) aufgetragen. Die ausgezogene Linie bezieht sich auf die linke Ordinate, und die gestrichelte Linie bezieht sich auf die rechte Ordinate.

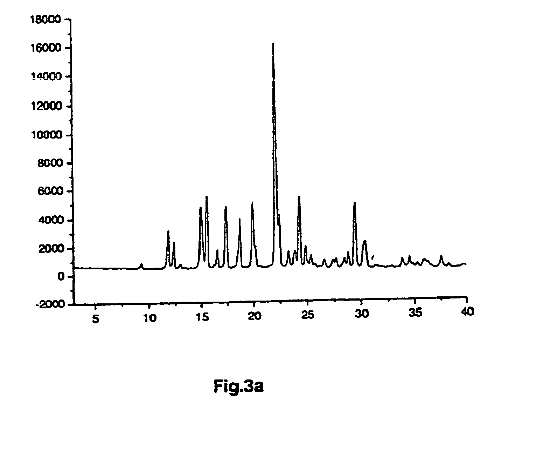

Figur 3a zeigt das Pulverröntgendiagramm der Verbindung der Formel IV. Auf der Abszisse sind die 2 Theta-Werte aufgetragen, und auf der Ordinate ist die Intensität (counts) aufgetragen.

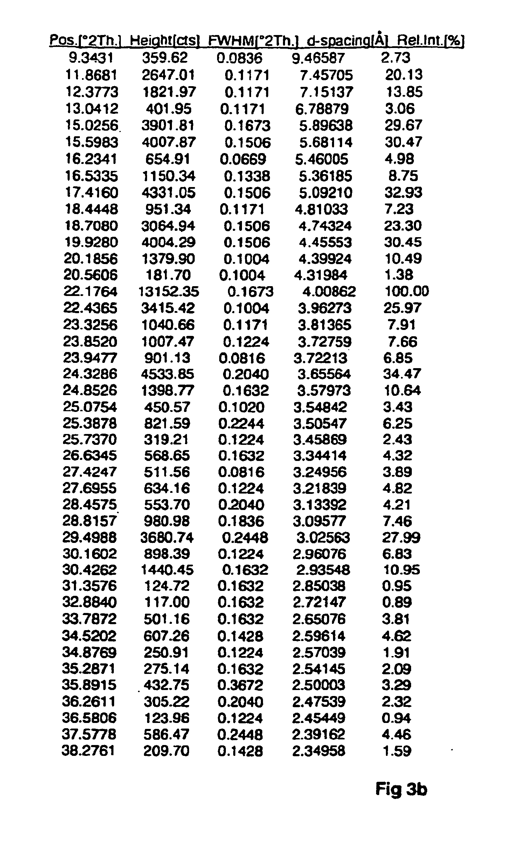

Figur 3b zeigt die 2 Theta-Werte mit einem Fehler von etwa ± 0,2 Grad des in Figur 3a gezeigten Pulverröntgendiagramms.

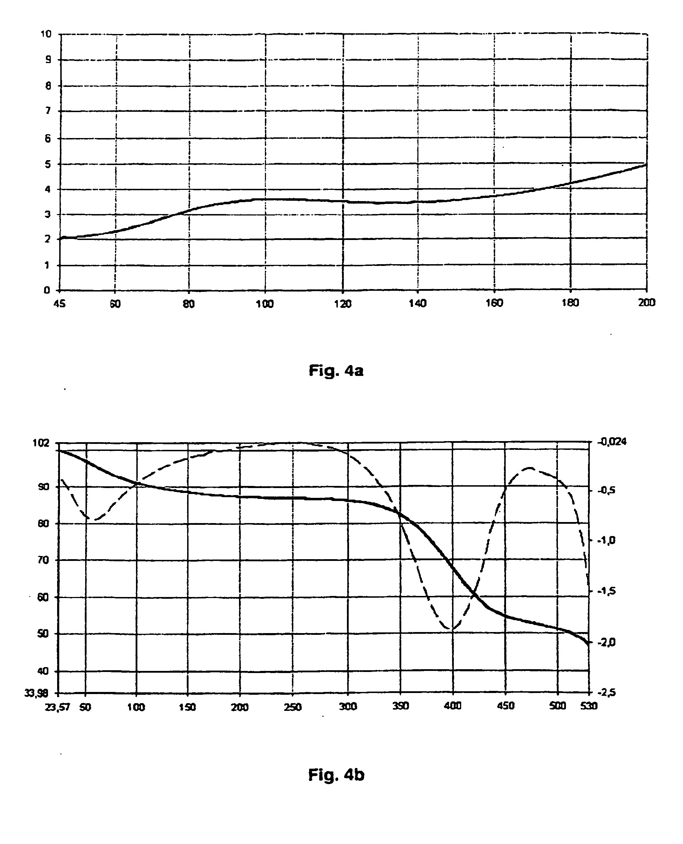

Figur 4a zeigt das "Differential Scanning Calorimetric" Profil, DSC, der Verbindung der Formel V. Auf der Abszisse ist die Temperatur in °C aufgetragen, und auf der Ordinate ist der endotherme Wärmefluss in mW aufgetragen.
Figur 4b zeigt das thermogravimetrische Profil, TGA, der Verbindung der Formel V. Auf der Abszisse ist die Temperatur in °C aufgetragen. Auf der linken Ordinate sind Gewichtsprozente und auf der rechten Ordinate ist die Ableitung dieser Gewichtsprozente gegenüber der Zeit t (Minuten) aufgetragen. Die ausgezogene Linie bezieht sich auf die linke Ordinate, und die gestrichelte Linie bezieht sich auf die rechte Ordinate.
Figur 5 zeigt das Pulverröntgendiagramm der Verbindung der Formel V. Auf der Abszisse sind die 2 Theta-Werte aufgetragen, und auf der Ordinate ist die Intensität (counts) aufgetragen.
Figur 6 zeigt das Raman Spektrum der Verbindung der Formel V. Auf der Abszisse sind die Wellenzahlen in cm⁻¹ aufgetragen, und auf der Ordinate ist die Raman Intensität aufgetragen.
(6S)-5,6,7,8-Tetrahydrofolsäure der Formel II mit einem Anteil des entsprechenden (6R)-Diastereo-isomers im Bereich von 4 Gew.-% bis 8 Gew.-% wurde gemäss EP 0 600 460 hergestellt.

Es war ganz überraschend, dass man eine wässrigen Lösung des Calciumsalzes von (6S)-N(5)-Methyl-5,6,7,8-tetrahydrofolsäure der Formel III mit einem erhöhten Gehalt des (6S)-Isomers erhalten konnte, und zwar durch selektives Auskristallisieren des 1:1 Gemisches der diastereoisomeren Salze aus der Lösung.

Routinemässig konnte man eine Lösung isolieren, welche einen Gehalt des (6S)-Isomers von wenigstens 98% hatte.

Es war auch ganz überraschend, die entsprechende stabile, kristalline freie Säure der Formel IV aus der oben beschriebenen Lösung zu erhalten: zudem wurde während dieses Schrittes eine weitere Erhöhung der diastereoisomeren Reinheit von wenigstens 99% erreicht.

Die stabile, kristalline (6S)-N(5)-Methyl-5,6,7,8-tetrahydrofolsäure der Formel IV kann in einer solchen diastereoisomeren Reinheit isoliert werden, dass diese Säure als wenigstens ein aktiver Bestandteil in einem Nährungsergänzungsmittel oder in einem Medikament verwendet werden kann.

Aus der Säure der Formel IV wird das überraschend stabile, amorphe Calciumsalzes der Formel V hergestellt.

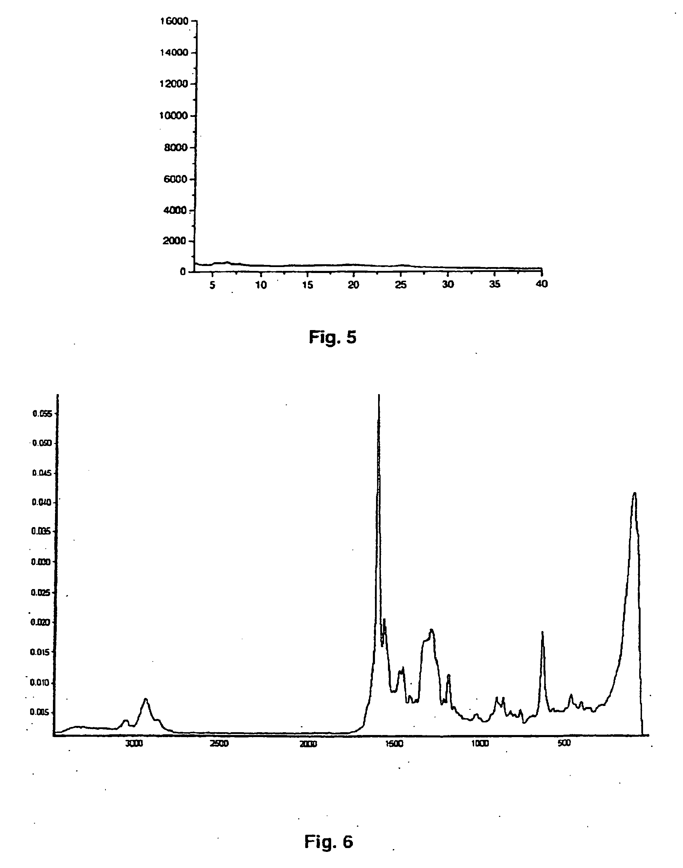

Aus dem Pulverröntgendiagramm der Verbindung der Formel V - siehe Figur 5 - geht hervor, dass diese Verbindung praktisch amorph ist.

Die vorliegende Erfindung wird durch die nachfolgenden Beispiele illustriert.

### Beispiel 1 (Herstellung der wässrige Lösung des Calciumsalzes von (6S)-N(5)-Methyl-5,6,7,8-tetrahydrofolsäure der Formel III)

100 g (6S)-5,6,7,8-Tetrahydrofolsäure der Formel II mit einer Diastereoisomerenreinheit von (6S):(6R) von 92:8 - hergestellt gemäss EP 0 600 460 - wurden in Wasser, welches eine Temperatur von 7°C hatte, unter einer Stickstoffatmosphäre suspendiert.

Die Verbindung der Formel II wurde durch die Hinzugabe von 65 ml 20%-iger (w/w) wässriger NaOH Lösung aufgelöst. Der pH-Wert betrug dann 9,05.

Zu dieser Lösung wurden 23,7 g 36%-ige (w/w) wässrige Formaldehydlösung innerhalb von 5 Minuten bei einer Temperatur von 8°C unter Rühren hinzugegeben.

Nach 15 Minuten wurde eine wässrige NaBH₄-Lösung, hergestellt durch Auflösen von 21,3 g NaBH₄ in 50 ml Wasser und 1 ml 20%-iger (w/w) wässriger NaOH Lösung, während 1 Stunde bei einer Temperatur von 8°C unter Rühren hinzugegeben.

Dieses Gemisch wurde während 30 Minuten bei einer Temperatur von 8°C und anschliessend während 20 Minuten bei einer Temperatur von 61°C gerührt.

Die Reaktionstemperatur wurde dann innerhalb von 2 Stunden auf 20°C erniedrigt.

Dann wurden 79 ml 18%-ige (w/w) wässriger HCl Lösung tropfenweise hinzugegeben. Der pH-Wert betrug dann 8,03.

Das Gemisch wurde auf eine Temperatur 4°C abgekühlt, um die Borat-Ausfällung zu ermöglichen. Nach 2 Stunden wurden die Borate durch Filtration entfernt.

Der pH-Wert der resultierenden Lösung wurde durch die Hinzugabe von 10 ml 18%-ige (w/w) wässriger HCl Lösung auf einen Wert von 7,03 eingestellt.

Diese Lösung wurde auf eine Temperatur von 20°C erwärmt, und dann wurden zuerst 1,62 g an Di-natrium-EDTA und dann 0,82 Äquivalente (25,44 g) an CaCl₂.2H₂O hinzugegeben.

Dann wurde der pH-Wert auf einen Wert von 6,9 durch die Hinzugabe von 2 ml 20%-iger (w/w) wässriger NaOH Lösung eingestellt.

Die selektive Kristallisation des Calciumsalzes von (6RS)-N(5)-Methyl-5,6,7,8-tetrahydrofolsäure erfolgte durch Animpfen der Lösung mit 100 mg von vorgängig hergestellten Kristallen des Calciumsalzes von (6RS)-N(5)-Methyl-5,6,7,8-tetrahydrofolsäure, gefolgt von einer Temperaturerniedrigung von Raumtemperatur auf eine Temperatur von 4°C innerhalb von 40 Minuten.

Die resultierende Suspension wurde unter Rühren während 18 Stunden bei einer Temperatur von 4°C gehalten.

Die erhaltenen Kristalle wurden abfiltriert.

Die so erhaltene wässrige Lösung des Calciumsalzes von (6S)-N(5)-Methyl-5,6,7,8-tetrahydrofolsäure der Formel III hatte einem Anteil des entsprechenden (6R)-Diastereoisomers von ≤ 2 Gew.-%, bestimmt mittels HPLC an einer chiralen Säule.

### Beispiel 2 (Herstellung von kristalliner (6S)-N(5)-Methyl-5,6,7,8-tetrahydrofolsäure der Formel IV)

Zur wässrigen Lösung des Calciumsalzes von (6S)-N(5)-Methyl-5,6,7,8-tetrahydrofolsäure der Formel III mit einem Anteil des entsprechenden (6R)-Diastereo-isomers von ≤ 2 Gew.-%, hergestellt gemäss obigem Beispiel 1, wurden innerhalb von 20 Minuten 6 ml 100% Essigsäure hinzugegeben, bis ein pH-Wert von 5,5 erhalten wurde.

Danach wurde die Temperatur auf 45°C erhöht.

Bei dieser Temperatur wurden nacheinander 1,35 g Natrium-dithionit und 1,9 g Di-natrium-EDTA hinzugegeben.

Zu dieser Lösung wurden 25 ml 100% Essigsäure innerhalb von 15 Minuten hinzugegeben.

Bei einem pH-Wert von 4,5 begann die Kristallisation von (6S)-N(5)-Methyl-5,6,7,8-tetrahydrofolsäure der Formel IV.

Der pH-Wert wurde im Bereich von 4,3 bis 4,4 durch kontinuierliche Hinzugabe von 100% Essigsäure (10 ml) gehalten.

Diese Suspension wurde 30 Minuten gerührt und anschliessend abfiltriert.

Der so erhaltene kristalline Festkörper wurde mit Wasser, welches eine Temperatur von 40°C hatte, gewaschen.

Es wurden 106,9 g an feuchtem kristallinen Festkörper erhalten.

Zur Bestimmung der Reinheit und des Verhältnisses der Diastereoisomeren wurde eine Probe der Verbindung der Formel IV mit 94%-igem (v/v) wässrigem Ethanol gewaschen und dann unter reduziertem Druck getrocknet. Dabei wurden folgende Resultate erhalten:
HPLC Reinheit: 96,45 %

Verhältnis von (6S)/(6R) - 99,1 : 0,9 (bestimmt mittels chiraler HPLC).

Das so erhaltene Produkt der Formel IV hatte die folgenden Stabilitätsdaten (gelagert bei einer Temperatur von 4°C):

| Zeit (Monate) | HPLC Reinheit (%) |
|---|---|
| 0 | 96.45 |
| 6 | 96.66 |

### Beispiel 3 (Herstellung des amorphen Calciumsalzes von (6S)-N(5)-Methyl-5,6,7,8-tetrahydrofolsäure der Formel V)

106,9 g des feuchten kristallinen Festkörpers, hergestellt gemäss obigem Beispiel 2, wurden in 400 ml Wasser, welches eine Temperatur von 40°C hatte suspendiert.

Zu dieser Suspension wurden 65 ml einer 20%-igen (w/w) wässrigen NaOH Lösung langsam hinzugegeben, bis eine klare Lösung mit einem pH-Wert von 6,8 erhalten wurde.

Zu dieser Lösung wurden 0,90 Äquivalente an CaCl₂.2H₂O (22,7 g, bezogen auf die Menge an isolierter trockener Verbindung der Formel IV) hinzugegeben.

Die HPLC Bestimmung von (6S)-N(5)-Methyl-5,6,7,8-tetrahydrofolsäure ergab eine Konzentration in Lösung von c = 14,7 % w/w.

Der pH-Wert wurde auf einen Wert von 6,8 durch die Hinzugabe von 1 ml 20%-iger (w/w) wässriger NaOH Lösung eingestellt.

Die Fällung des Calciumsalzes von (6S)-N(5)-Methyl-5,6,7,8-tetrahydrofolsäure der Formel V wurde durch die Hinzugabe von 100 mg an vorgängig hergestellter Verbindung der Formel V gestartet.

Das so hergestellte Gemisch wurde während 1 Stunde bei einer Temperatur von 40°C gehalten.

Danach wurde innerhalb von 2 Stunden die Temperatur von 40°C auf eine Temperatur von 23°C gesenkt, und die Temperatur von 23°C wurde während 18 Stunden aufrecht erhalten.

Wenn die Konzentration des Calciumsalzes von (6S)-N(5)-Methyl-5,6,7,8-tetrahydrofolsäure der Formel V in der Mutterlauge unter 7,5 % war, wurde die Suspension abfiltriert.

Der erhaltene Festkörper wurde mit 50 ml Wasser, welches eine Temperatur von 10°C hatte, gewaschen.

Die nasse Verbindung der Formel V wurde in 250 ml mit 94%-igem (v/v) wässrigem Ethanol bei einer Temperatur von 22°C suspendiert und während 30 Minuten gerührt.

Dann wurde diese Suspension abfiltriert und zweimal mit 50 ml 94%-igem (v/v) wässrigem Ethanol gewaschen.

Man erhielt 95 g an nassem Festkörper, welcher unter reduziertem Druck getrocknet wurde. Man erhielt 47,3 g der Verbindung der Formel V.

Dabei wurden folgende analytische Daten erhalten:
HPLC Reinheit: 98,75 %

Verhältnis von (6S) / (6R) = 99,75 : 0,25 (bestimmt mittels chiraler HPLC).

Das so erhaltene Produkt der Formel V hatte die folgenden Stabilitätsdaten (gelagert bei einer Temperatur von 25°C unter Vakuum [2 mbar]):

| Zeit (Monate) | HPLC Assay (%) | HPLC Reinheit (%) |
|---|---|---|
| 0 | 99,0 | 98,7 |
| 1 | 98,8 | 98,7 |
| 2 | 99,8 | 98,9 |
| 3 | 100,2 | 98,8 |
| 6 | 99,9 | 98,5 |
| 9 | 100,7 | 98,9 |

## Patentansprüche

1. Verfahren zur Herstellung einer wässrigen Lösung des Calciumsalzes von (6S)-N(5)-Methyl-5,6,7,8-tetrahydrofolsäure,
wobei man (6S)-5,6,7,8-Tetrahydrofolsäure mit einem Anteil des entsprechenden (6R)-Diastereoisomers im Bereich von 4 Gew.-% bis 8 Gew.-% in Wasser methyliert,
**dadurch gekennzeichnet, dass** man
- zum erhaltenen methylierten Reaktionsgemisch von 0,70 bis 0,82 Äquivalente, bezogen auf die eingesetzte Menge an THF, an Calciumchlorid hinzugibt,
- aus der erhaltenen wässrigen Lösung das Calciumsalz von (6RS)-N(5)-Methyl-5,6,7,8-tetrahydrofolsäure selektiv auskristallisiert und abtrennt, und
- die wässrige Lösung des Calciumsalzes von (6S)-N(5)-Methyl-5,6,7,8-tetrahydrofolsäure mit einem Anteil des entsprechenden (6R)-Diastereoisomers von ≤ 2 Gew.-% gewinnt.

2. Verfahren nach Anspruch 1, **dadurch gekennzeichnet, dass** das Calciumchlorid in fester Form oder in der Form einer wässrigen Lösung hinzugegeben wird.

3. Verfahren nach einem der Ansprüche 1 bis 2, **dadurch gekennzeichnet, dass** die selektive Kristallisation des Calciumsalzes von (6RS)-N(5)-Methyl-5,6,7,8-tetrahydrofolsäure durch Animpfen mit vorgängig hergestellten Kristallen des Calciumsalzes von (6RS)-N(5)-Methyl-5,6,7,8-tetrahydrofolsäure erfolgt, gefolgt von einer Temperaturerniedrigung von Raumtemperatur auf eine Temperatur im Bereich von 3°C bis 5°C, und dass diese Temperatur während 16 bis 18 Stunden aufrecht gehalten wird.

4. Verfahren zur Herstellung des amorphen Calciumsalzes von (6S)-N(S)-Methyl-5,6,7,8-tetrahydrofolsäure
**dadurch gekennzeichnet, dass** man
- kristalline (6S)-N(S)-Methyl-5,6,7,8-tetra-hydrofolsäure in Wasser suspendiert, wobei das Wasser eine Temperatur von 35°C bis 41°C hat,
wobei man die kristalline (6S)-N(5)-Methyl-5,6,7,8-tetrahydrofolsäure gemäss einem Verfahren herstellt, bei dem man
- - zu einer wässrigen Lösung des Calciumsalzes von (6S)-N(5)-Methyl-5,6,7,8-tetrahydrofolsäure mit einem Anteil des entsprechenden (6R)-Diastereoisomers von ≤ 2 Gew.-% entweder Essigsäure oder eine Sulfonsäure in einer solchen Menge hinzugibt, bis ein pH-Wert von 5,5 erhalten wird,
- - die so erhaltene Lösung auf eine Temperatur im Bereich von 44°C bis 46°C erwärmt,
- - zu dieser erwärmten Lösung entweder Essigsäure oder eine Sulfonsäure in einer solchen Menge hinzugibt, bis ein pH-Wert im Bereich von 4,3 bis 4,4 erhalten wird, wobei die Kristallisation von (6S)-N(5)-Methyl-5,6,7,8-tetrahydrofolsäure beginnt, wobei während dieser Kristallisation der pH-Wert im Bereich von 4,3 bis 4,4 durch kontinuierliche Hinzugabe von entweder Essigsäure oder einer Sulfonsäure aufrecht erhalten wird, und
- - den so erhaltenen kristallinen Festkörper bei einer Temperatur im Bereich von 44°C bis 46°C abfiltriert und isoliert,
- zu dieser Suspension eine NaOH Lösung in einer solchen Menge portionenweise hinzugibt, bis ein pH-Wert im Bereich von 6,7 bis 6,9 erhalten wird,
- zur so hergestellten Lösung 0,90 Äquivalente an Calciumchlorid, bezogen auf die eingesetzte Menge an kristalliner (6S)-N(5)-Methyl-5,6,7,8-tetrahydrofolsäure, hinzugibt,
- in der so hergestellten Lösung die Konzentration an kristalliner (6S)-N(5)-Methyl-5,6,7,8-tetrahydrofolsäure entweder durch Hinzugabe oder durch Entfernung von Wasser auf einen Wert im Bereich von 14 Gew.-% bis 16 Gew.-% einstellt,
- die Fällung des Calciumsalzes von (6S)-N(5)-Methyl-5,6,7,8-tetrahydrofolsäure durch die Hinzugabe einer kleinen Menge an vorgängig hergestelltem Calciumsalz von (6S)-N(5)-Methyl-5,6,7,8-tetrahydrofolsäure startet,
- das so hergestellte Gemisch während 1 Stunde bei einer Temperatur von 40°C hält,
- danach innerhalb von 2 Stunden die Temperatur von 40°C auf eine Temperatur von 23°C senkt,
- die genannte Temperatur von 23°C während 16 bis 18 Stunden aufrecht erhält, und
- den erhaltenen Festkörper isoliert.

5. Verfahren nach Anspruch 4, **dadurch gekennzeichnet, dass** man den erhaltenen kristallinen Festkörper mit Wasser, welches eine Temperatur von 40°C hat, wäscht.

6. Verfahren nach einem der Ansprüche 4 bis 5, **dadurch gekennzeichnet, dass** die Sulfonsäure p-Toluolsulfonsäure ist.

7. Verfahren nach einem der Ansprüche 4 bis 6, **dadurch gekennzeichnet, dass** man die wässrige Lösung des Calciumsalzes von (6S)-N(5)-Methyl-5,6,7,8-tetrahydrofolsäure mit einem Anteil des entsprechenden (6R)-Diastereoisomers von ≤ 2 Gew.-% gemäss dem Verfahren nach einem der Ansprüche 1 bis 3 herstellt.

8. Verfahren nach Anspruch 4, **dadurch gekennzeichnet, dass** man den erhaltenen Festkörper mit Wasser, welches eine Temperatur von 10°C hat, wäscht, den gewaschenen Festkörper in 94%-igem wässrigen Ethanol bei Raumtemperatur suspendiert und anschliessend den so behandelten Festkörper mittels Filtration isoliert.

9. Verfahren nach einem der Ansprüche 4 oder 8, **dadurch gekennzeichnet, dass** das Calciumchlorid in fester Form oder in der Form einer wässrigen Lösung hinzugegeben wird.

10. Amorphes Calciumsalz von (6S)-N(5)-Methyl-5,6,7,8-tetrahydrofolsäure
dadurch erhältlich, dass man
- kristalline (6S)-N(5)-Methyl-5,6,7,8-tetrahydrofolsäure in Wasser suspendiert, wobei das Wasser eine Temperatur von 35°C bis 41°C hat,
wobei man die kristalline (6S)-N(5)-Methyl-5,6,7,8-tetrahydrofolsäure gemäss einem Verfahren herstellt, bei dem man
- - zu einer wässrigen Lösung des Calciumsalzes von (6S)-N(5)-Methyl-5,6,7,8-tetrahydrofolsäure mit einem Anteil des entsprechenden (6R)-Diastereoisomers von ≤ 2 Gew.-% entweder Essigsäure oder eine Sulfonsäure in einer solchen Menge hinzugibt, bis ein pH-Wert von 5,5 erhalten wird,
- - die so erhaltene Lösung auf eine Temperatur im Bereich von 44°C bis 46°C erwärmt,
- - zu dieser erwärmten Lösung entweder Essigsäure oder eine Sulfonsäure in einer solchen Menge hinzugibt, bis ein pH-Wert im Bereich von 4,3 bis 4,4 erhalten wird, wobei die Kristallisation von (6S)-N(5)-Methyl-5,6,7,8-tetrahydrofolsäure beginnt, wobei während dieser Kristallisation der pH-Wert im Bereich von 4,3 bis 4,4 durch kontinuierliche Hinzugabe von entweder Essigsäure oder einer Sulfonsäure aufrecht erhalten wird, und
- - den so erhaltenen kristallinen Festkörper bei einer Temperatur im Bereich von 44°C bis 46°C abfiltriert und isoliert,
- zu dieser Suspension eine NaOH Lösung in einer solchen Menge portionenweise hinzugibt, bis ein pH-Wert im Bereich von 6,7 bis 6,9 erhalten wird,
- zur so hergestellten Lösung 0,90 Äquivalente an Calciumchlorid, bezogen auf die eingesetzte Menge an kristalliner (6S)-N(5)-Methyl-5,6,7,8-tetrahydrofolsäure, hinzugibt,
- in der so hergestellten Lösung die Konzentration an kristalliner (6S)-N(5)-Methyl-5,6,7,8-tetrahydrofolsäure entweder durch Hinzugabe oder durch Entfernung von Wasser auf einen Wert im Bereich von 14 Gew.-% bis 16 Gew.-% einstellt,
- die Fällung des Calciumsalzes von (6S)-N(5)-Methyl-5,6,7,8-tetrahydrofolsäure durch die Hinzugabe einer kleinen Menge an vorgängig hergestelltem Calciumsalz von (6S)-N(5)-Methyl-5,6,7,8-tetrahydrofolsäure startet,
- das so hergestellte Gemisch während 1 Stunde bei einer Temperatur von 40°C hält,
- danach innerhalb von 2 Stunden die Temperatur von 40°C auf eine Temperatur von 23°C senkt,
- die genannte Temperatur von 23°C während 16 bis 18 Stunden aufrecht erhält, und
- den erhaltenen Festkörper isoliert.

11. Amorphes Calciumsalz von (6S)-N(5)-Methyl-5,6,7,8-tetrahydrofolsäure nach Anspruch 10,
dadurch erhältlich, dass man das Verfahren gemäss einem der Ansprüche 8 bis 9 durchführt.

12. Amorphes Calciumsalz von (6S)-N(5)-Methyl-5,6,7,8-tetrahydrofolsäure nach einem der Ansprüche 10 bis 11, **dadurch gekennzeichnet, dass** es
- das in Figur 4a gezeigte "Differential Scanning Calorimetric" Profil, DSC,
- das in Figur 4b gezeigte das thermogravimetrische Profil, TGA,
- das in Figur 5 gezeigte Pulverröntgendiagramm, und
- das in Figur 6 gezeigte Raman Spektrum hat.

## Claims

1. A process for the preparation of an aqueous solution of the calcium salt of (6S)-N(5)-methyl-5,6,7,8-tetrahydrofolic acid,
whereby (6S)-5,6,7,8-tetrahydrofolic acid having a content of the corresponding (6R)-diastereoisomer in the range from 4% by weight to 8% by weight is methylated in water,
**characterized in that**
- to the obtained methylated reaction mixture are added from 0.70 to 0.82 equivalents, referred to the used amount of THF, of calcium chloride,
- from the so obtained aqueous solution the calcium salt of (6RS)-N(5)-methyl-5,6,7,8-tetrahydrofolic acid is selectively crystallized and separated, and
- the aqueous solution of the calcium salt of (6S)-N(5)-methyl-5,6,7,8-tetrahydrofolic acid having a content of the corresponding (6R)-diastereoisomer of ≤ 2% by weight is obtained.

2. The process according to claim 1, **characterized in that** the calcium chloride is added in solid form or in the form of an aqueous solution.

3. The process according to one of claims 1 to 2, **characterized in that** selective crystallization of the calcium salt of (6RS)-N(5)-methyl-5,6,7,8-tetrahydrofolic acid is realized by seeding with previously prepared crystals of the calcium salt of (6RS)-N(5)-methyl-5,6,7,8-tetrahydrofolic acid, followed by a lowering of the temperature from room temperature to a temperature in the range from 3°C to 5°C, and that this temperature is kept during 16 to 18 hours.

4. A process for the preparation of the amorphous calcium salt of (6S)-N(5)-methyl-5,6,7,8-tetrahydrofolic acid,
**characterized in that**
- crystalline (6S)-N(5)-methyl-5,6,7,8-tetrahydrofolic acid is suspended in water, whereby the water has a temperature from 35°C to 41°C,
whereby the crystalline (6S)-N(5)-methyl-5,6,7,8-tetrahydrofolic acid is prepared according to a process wherein
- - to an aqueous solution of the calcium salt of (6S)-N(5)-methyl-5,6,7,8-tetrahydrofolic acid having a content of the corresponding (6R)-diastereoisomer of ≤ 2% by weight is added either acetic acid or a sulfonic acid in such an amount, until a pH-value of 5.5 is obtained,
- - the so obtained solution is warmed to a temperature in the range from 44°C to 46°C,
- - to this warmed solution is added either acetic acid or a sulfonic acid in such an amount, until a pH-value in the range from 4.3 to 4.4 is obtained, whereby the crystallization of (6S)-N(5)-methyl-5,6,7,8-tetrahydrofolic acid begins, whereby during this crystallization the pH-value is maintained in the range from 4.3 to 4.4 by continuous addition of either acetic acid or of a sulfonic acid, and
- - the so obtained crystalline solid is filtered off at a temperature in the range from 44°C to 46°C and is isolated,
- to this suspension is added a NaOH solution in portions and in such an amount until a pH-value in the range from 6.7 to 6.9 is obtained,
- to the so prepared solution are added 0.90 equivalents of calcium chloride, referred to the used amount of crystalline (6S)-N(5)-methyl-5,6,7,8-tetrahydro-folic acid,
- in the so prepared solution the concentration of crystalline (6S)-N(5)-methyl-5,6,7,8-tetrahydrofolic acid is adjusted either by the addition or by the removal of water to a value in the range from 14% by weight to 16% by weight,
- the precipitation of the calcium salt of (6S)-N(5)-methyl-5,6,7,8-tetrahydrofolic acid is started by the addition of a small amount of previously prepared calcium salt of (6S)-N(5)-methyl-5,6,7,8-tetrahydrofolic acid,
- the so prepared mixture is kept during 1 hour at a temperature of 40°C,
- then within 2 hours the temperature is lowered from 40°C to a temperature of 23°C,
- said temperature of 23°C is maintained during 16 to 18 hours, and
- the obtained solid is isolated.

5. The process according to claim 4, **characterized in that** the obtained crystalline solid is washed with water having a temperature of 40°C.

6. The process according to one of claims 4 to 5, **characterized in that** the sulfonic acid is p-toluene sulfonic acid.

7. The process according to one of claims 4 to 6, **characterized in that** the aqueous solution of the calcium salt of (6S)-N(5)-methyl-5,6,7,8-tetrahydrofolic acid having a content of the corresponding (6R)-diastereoisomer of ≤ 2% by weight is prepared according to the process according to one of claims 1 to 3.

8. The process according to claim 4, **characterized in that** the obtained solid is washed with water having a temperature of 10°C, the washed solid is suspended at room temperature in 94% aqueous ethanol and then the so treated solid is isolated by means of filtration.

9. The process according to one of claim 4 or 8, **characterized in that** the calcium chloride is added in solid form or in the form of an aqueous solution.

10. Amorphous calcium salt of (6S)-N(5)-methyl-5,6,7,8-tetrahydrofolic acid,
obtainable in that
- crystalline (6S)-N(5)-methyl-5,6,7,8-tetrahydrofolic acid is suspended in water, whereby the water has a temperature from 35°C to 41°C,
whereby the crystalline (6S)-N(5)-methyl-5,6,7,8-tetrahydrofolic acid is prepared according to a process wherein
- - to an aqueous solution of the calcium salt of (6S)-N(5)-methyl-5,6,7,8-tetrahydrofolic acid having a content of the corresponding (6R)-diastereoisomer of ≤ 2% by weight is added either acetic acid or a sulfonic acid in such an amount, until a pH-value of 5.5 is obtained,
- - the so obtained solution is warmed to a temperature in the range from 44°C to 46°C,
- - to this warmed solution is added either acetic acid or a sulfonic acid in such an amount, until a pH-value in the range from 4.3 to 4.4 is obtained, whereby the crystallization of (6S)-N(5)-methyl-5,6,7,8-tetrahydrofolic acid begins, whereby during this crystallization the pH-value is maintained in the range from 4.3 to 4.4 by continuous addition of either acetic acid or of a sulfonic acid, and
- - the so obtained crystalline solid is filtered off at a temperature in the range from 44°C to 46°C and is isolated,
- to this suspension is added a NaOH solution in portions and in such an amount until a pH-value in the range from 6.7 to 6.9 is obtained,
- to the so prepared solution are added 0.90 equivalents of calcium chloride, referred to the used amount of crystalline (6S)-N(5)-methyl-5,6,7,8-tetrahydro-folic acid,
- in the so prepared solution the concentration of crystalline (6S)-N(5)-methyl-5,6,7,8-tetrahydrofolic acid is adjusted either by the addition or by the removal of water to a value in the range from 14% by weight to 16% by weight,
- the precipitation of the calcium salt of (6S)-N(5)-methyl-5,6,7,8-tetrahydrofolic acid is started by the addition of a small amount of previously prepared calcium salt of (6S)-N(5)-methyl-5,6,7,8-tetrahydrofolic acid,
- the so prepared mixture is kept during 1 hour at a temperature of 40°C,
- then within 2 hours the temperature is lowered from 40°C to a temperature of 23°C,
- said temperature of 23°C is maintained during 16 to 18 hours, and
- the obtained solid is isolated.

11. Amorphous calcium salt of (6S)-N(5)-methyl-5,6,7,8-tetrahydrofolic acid according to claim 10,
obtainable in that the process according to one of claims 8 to 9 is carried out.

12. Amorphous calcium salt of (6S)-N(5)-methyl-5,6,7,8-tetrahydrofolic acid according to one of claims 10 to 11, **characterized in that** it has
- the in Figure 4a shown "differential scanning calorimetric" profile, DSC,
- the in Figure 4b shown thermo gravimetric profile, TGA,
- the in Figure 5 shown X-ray powder diffraction diagram, and
- the in Figure 6 shown Raman spectrum.

## Revendications

1. Procédé de préparation d'une solution aqueuse du sel de calcium de l'acide (6S)-N(5)-méthyl-5,6,7,8-tétrahydrofolique, dans lequel
on méthyle dans de l'eau l'acide (6S)-5,6,7,8-tétrahydrofolique présentant une proportion du diastéréoisomère (6R) correspondant comprise dans l'intervalle de 4 à 8 % en poids,
**caractérisé en ce qu'**on
- ajoute au mélange réactionnel méthylé obtenu de 0,70 à 0,82 équivalent de chlorure de calcium par rapport à la quantité de THF utilisée,
- cristallise sélectivement et sépare, à partir de la solution aqueuse obtenue, le sel de calcium de l'acide (6RS)-N(5)-méthyl-5,6,7,8-tétrahydrofolique et
- extrait la solution aqueuse du sel de calcium de l'acide (6S)-N(5)-méthyl-5,6,7,8-tétrahydrofolique présentant une proportion du diastéréoisomère (6R) correspondant inférieure ou égale à 2 % en poids.

2. Procédé selon la revendication 1, **caractérisé en ce que** le chlorure de calcium est ajouté sous forme solide ou sous forme d'une solution aqueuse.

3. Procédé selon l'une des revendications 1 à 2, **caractérisé en ce que** la cristallisation sélective du sel de calcium de l'acide (6RS)-N(5)-méthyl-5,6,7,8-tétrahydrofolique est opérée par ensemencement avec des cristaux préalablement préparés de sel de calcium de l'acide (6RS)-N(5)-méthyl-5,6,7,8-tétrahydrofolique, suivi d'un abaissement de la température, passant de la température ambiante à une température située dans l'intervalle de 3 à 5 °C, et **en ce que** cette température est maintenue pendant 16 à 18 heures.

4. Procédé de préparation du sel de calcium amorphe de l'acide (6S)-N(5)-méthyl-5,6,7,8-tétrahydrofolique,
**caractérisé en ce qu'**on
- met en suspension dans de l'eau l'acide (6S)-N(5)-méthyl-5,6,7,8-tétrahydrofolique cristallin, l'eau présentant une température de 35 °C à 41 °C,
- l'acide (6S)-N(5)-méthyl-5,6,7,8-tétrahydrofolique cristallin étant préparé selon un procédé dans lequel on
- - ajoute à une solution aqueuse du sel de calcium de l'acide (6S)-N(5)-méthyl-5,6,7,8-tétrahydrofolique présentant une proportion inférieure ou égale à 2 % en poids du diastéréoisomère (6R) correspondant, soit de l'acide acétique, soit un acide sulfonique, dans une proportion telle qu'elle permette d'obtenir un pH de 5,5,
- - chauffe la solution ainsi obtenue à une température située dans l'intervalle de 44 °C à 46 °C,
- - ajoute à cette solution chauffée, soit de l'acide acétique, soit un acide sulfonique dans une proportion telle qu'elle permette d'obtenir un pH compris dans l'intervalle de 4,3 à 4,4 auquel la cristallisation de l'acide (6S)-N(5)-méthyl-5,6,7,8-tétrahydrofolique commence, ce pH situé dans l'intervalle de 4,3 à 4,4 étant maintenu durant cette cristallisation par l'adjonction continue, soit d'acide acétique, soit d'un acide sulfonique, et
- - sépare par filtration et isole le solide cristallin ainsi obtenu à une température située dans l'intervalle de 44 °C à 46 °C,
- ajoute par portions à cette suspension une solution de NaOH dans une proportion telle qu'elle permette d'obtenir un pH situé dans l'intervalle de 6,7 à 6,9,
- ajoute à la solution ainsi préparée, 0,90 équivalent de chlorure de calcium par rapport à la proportion mise en oeuvre d'acide (6S)-N(5)-méthyl-5,6,7,8-tétrahydrofolique cristallin,
- ajuste dans la solution ainsi préparée la concentration de l'acide (6S)-N(5)-méthyl-5,6,7,8-tétrahydrofolique cristallin à une valeur située dans l'intervalle de 14 à 16 % en poids, soit par adjonction, soit par élimination d'eau,
- amorce la précipitation du sel de calcium de l'acide (6S)-N(5)-méthyl-5,6,7,8-tétrahydrofolique par l'adjonction d'une petite proportion du sel de calcium de l'acide (6S)-N(5)-méthyl-5,6,7,8-tétrahydrofolique préalablement préparé,
- maintient le mélange ainsi préparé pendant 1 heure à une température de 40 °C,
- abaisse ensuite endéans 2 heures de temps la température de 40 °C à une température de 23 °C,
- maintient la température mentionnée de 23 °C pendant 16 à 18 heures et
- isole le solide obtenu.

5. Procédé selon la revendication 4, **caractérisé en ce qu'**on lave le solide cristallin obtenu avec de l'eau présentant une température de 40 °C.

6. Procédé selon l'une des revendications 4 à 5, **caractérisé en ce que** l'acide sulfonique est l'acide p-toluène sulfonique.

7. Procédé selon l'une des revendications 4 à 6, **caractérisé en ce qu'**on prépare la solution aqueuse du sel de calcium de l'acide (6S)-N(5)-méthyl-5,6,7,8-tétrahydrofolique présentant une proportion du diastéréoisomère (6R) correspondant inférieure ou égale à 2 % en poids, conformément au procédé selon l'une des revendications 1 à 3.

8. Procédé selon la revendication 4, **caractérisé en ce qu'**on lave le solide obtenu avec de l'eau présentant une température de 10°C, met le solide lavé en suspension dans de l'éthanol aqueux à 94 % à température ambiante et isole ensuite le solide ainsi traité par filtration.

9. Procédé selon l'une des revendications 4 ou 8, **caractérisé en ce que** le chlorure de calcium est ajouté sous forme solide ou sous forme d'une solution aqueuse.

10. Sel de calcium amorphe de l'acide (6S)-N(5)-méthyl-5,6,7,8-tétrahydrofolique
obtenable en
- mettant en suspension dans de l'eau l'acide (6S)-N(5)-méthyl-5,6,7,8-tétrahydrofolique cristallin, l'eau présentant une température de 35 °C à 41 °C,
l'acide (6S)-N(5)-méthyl-5,6,7,8-tétrahydrofolique cristallin étant préparé selon un procédé dans lequel on
- - ajoute à une solution aqueuse du sel de calcium de l'acide (6S)-N(5)-méthyl-5,6,7,8-tétrahydrofolique présentant une proportion inférieure ou égale à 2 % en poids du diastéréoisomère (6R) correspondant, soit de l'acide acétique, soit un acide sulfonique, dans une proportion telle qu'elle permette d'obtenir un pH de 5,5,
- - chauffe la solution ainsi obtenue à une température située dans l'intervalle de 44 °C à 46 °C,
- - ajoute à cette solution chauffée, soit de l'acide acétique, soit un acide sulfonique dans une proportion telle qu'elle permette d'obtenir un pH compris dans l'intervalle de 4,3 à 4,4 auquel la cristallisation de l'acide (6S)-N(5)-méthyl-5,6,7,8-tétrahydrofolique commence, ce pH situé dans l'intervalle de 4,3 à 4,4 étant maintenu durant cette cristallisation par l'adjonction continue, soit d'acide acétique, soit d'un acide sulfonique, et
- - sépare par filtration et isole le solide cristallin ainsi obtenu à une température située dans l'intervalle de 44 °C à 46 °C,
- ajoute par portions à cette suspension une solution de NaOH dans une proportion telle qu'elle permette d'obtenir un pH situé dans l'intervalle de 6,7 à 6,9,
- ajoute à la solution ainsi préparée, 0,90 équivalent de chlorure de calcium par rapport à la proportion mise en oeuvre d'acide (6S)-N(5)-méthyl-5,6,7,8-tétrahydrofolique cristallin,
- ajuste dans la solution ainsi préparée la concentration de l'acide (6S)-N(5)-méthyl-5,6,7,8-tétrahydrofolique cristallin à une valeur située dans l'intervalle de 14 à 16 % en poids, soit par adjonction, soit par élimination d'eau,
- amorce la précipitation du sel de calcium de l'acide (6S)-N(5)-méthyl-5,6,7,8-tétrahydrofolique par l'adjonction d'une petite proportion du sel de calcium de l'acide (6S)-N(5)-méthyl-5,6,7,8-tétrahydrofolique préalablement préparé,
- maintient le mélange ainsi préparé pendant 1 heure à une température de 40 °C,
- abaisse ensuite endéans 2 heures de temps la température de 40 °C à une température de 23 °C,
- maintient la température mentionnée de 23 °C pendant 16 à 18 heures et
- isole le solide obtenu.

11. Sel de calcium amorphe de l'acide (6S)-N(5)-méthyl-5,6,7,8-tétrahydrofolique selon la revendication 10,
obtenable en exécutant le procédé selon l'une des revendications 8 à 9.

12. Sel de calcium amorphe de l'acide (6S)-N(5)-méthyl-5,6,7,8-tétrahydrofolique selon l'une des revendications 10 à 11, **caractérisé en ce qu'**il possède
- le profil "Differential Scanning Calorimetric", DSC, représenté à la figure 4a,
- le profil thermogravimétrique, TGA, représenté à la figure 4b,
- le diagramme de diffraction des rayons X sur poudre, représenté à la figure 5,
- le spectre Raman représenté à la figure 6.
